# EUROPÄISCHE PATENTANMELDUNG

(11) **EP 2 995 328 A1**
(43) Veröffentlichungstag der Anmeldung: **16.03.2016**
(21) Anmeldenummer: 15184502.1
(22) Anmeldetag: 09.09.2015
(51) Int. Cl.: A61M 1/16, A61J 1/14

(54) **BEHÄLTER ZUR HERSTELLUNG VON DIALYSEKONZENTRATEN**

(30) Priorität: 09.09.2014 DE 202014104252 U
(71) Anmelder: Nephtec GmbH, 63477 Maintal (DE)
(72) Erfinder: Schwepe, Holger, 63477 Maintal (DE); Koukol, Robert, 63450 Hanau (DE)
(74) Vertreter: Stoffregen, Hans-Herbert

(57) **Zusammenfassung**

Die Erfindung bezieht sich auf einen Behälter (10), insbesondere wiederverwendbaren Mischbehälter, zur Herstellung von Dialysekonzentraten, wobei der Behälter (10) zur Herstellung eines Dialysekonzentrates benötigte Stoffe mit Ausnahme von Wasser enthält, umfassend ein vorzugsweise integriertes Steigrohr (26) zum Zuführen und Absaugen von Flüssigkeit, wobei sich das Steigrohr (26) von einem Schlauchanschluss (28) bis zu einem Boden (16) des Behälters (10) erstreckt und zumindest eine seitliche Öffnung (32) in einem bodenseitigen Ende (30) aufweist. Um einen Betrieb des Behälters auch bei einer Verstopfung der seitlichen Öffnung wegen Verklumpung der Stoffe zu ermöglichen, ist vorgesehen, dass das Steigrohr (26) zumindest eine Freispülöffnung (38) zur Erzeugung einer Flüssigkeitsströmung zur Auflösung seiner die zumindest eine seitliche Öffnung verstopfenden Verklumpung von Stoffen aufweist.

## Beschreibung

Die Erfindung bezieht sich auf einen Behälter, insbesondere wiederverwendbaren Mischbehälter, zur Herstellung von Dialysekonzentraten, wobei der Behälter zur Herstellung eines Dialysekonzentrates benötigte Stoffe mit Ausnahme von Wasser enthält, umfassend ein vorzugsweise integriertes Steigrohr zum Zuführen und Absaugen von Flüssigkeit, wobei sich das Steigrohr von einem Schlauchanschluss bis zu einem Boden des Behälters erstreckt und zumindest eine seitliche Öffnung in seinem bodenseitigen Ende aufweist.

Ein Behälter zur Herstellung von Dialysekonzentraten der eingangs genannten Art ist in der DE 103 13 965 B3 beschrieben. Bei dem bekannten Behälter weist das Steigrohr in Bodennähe eine seitliche Abgangsöffnung auf, über die Flüssigkeit während der Produktion der Salzlösung aus dem Steigrohr ausströmt und so in eine Drehbewegung versetzt wird.

Beim Einfüllen und Lagern der zur Herstellung des Dialysekonzentrats benötigten Stoffe ist nicht ausgeschlossen, dass die Stoffe im Bereich der seitlichen Ausgangsöffnung verklumpen, was dazu führt, dass die seitliche Öffnung verstopfen könnte. Folglich ist ein Einströmen von Wasser zur Herstellung des Konzentrats behindert.

Davon ausgehend liegt der vorliegenden Erfindung die Aufgabe zu Grunde, einen Behälter der eingangs genannten Art derart weiterzubilden, dass ein Betrieb des Behälters auch bei einer Verstopfung der seitlichen Öffnung ermöglicht wird, die durch zur Herstellung des Dialysekonzentrats benötigter Stoffe verursacht ist.

Das Problem wird erfindungsgemäß u. a. dadurch gelöst, dass das Steigrohr zumindest eine Freispülöffnung zur Erzeugung einer Flüssigkeitsströmung zur Auflösung einer die zumindest eine seitliche Öffnung verstopfenden Verklumpung von Stoffen aufweist. Gegenüber dem Stand der Technik wird der Vorteil erreicht, dass bei Verstopfung der seitlichen Öffnung z. B. durch Verklumpung von Salzen ein Freispülen der seitlichen Öffnung durch Auflösen der Verklumpung ermöglicht wird.

Gemäß einer bevorzugten Ausführungsform ist vorgesehen, dass die zumindest eine Freispülöffnung in dem bodenseitigen Ende angeordnet und in einen zwischen bodenseitigem Ende und dem Boden gebildeten Freiraum ausgerichtet ist.

Das bodenseitige Ende ist unter Bildung des Freiraums in Form eines Spaltes über dem Boden angeordnet, wobei die Freispülöffnung in den zwischen dem bodenseitigen Ende und dem Boden gebildeten, strömungstechnisch mit der zumindest einen seitlichen Öffnung gekoppelten Spalt als Freispülkanal gerichtet ist.

Die Stoffe werden von oben in das Behältnis gefüllt, wobei unterhalb des Steigrohrs aufgrund der geringen Spaltbildung zwischen dem bodenseitigen Ende des Steigrohrs und dem Boden des Behältnisses Stoffe nicht abgelagert werden können. Dadurch wird eine Schattenbildung unterhalb des Steigrohres erreicht, d. h., es wird vermieden, dass zur Herstellung des Dialysekonzentrats benötigte Stoffe sich unterhalb des Steigrohres ansammeln. Der Spalt und somit der Freispülkanal ist frei von Stoffen, so dass über die Freispülöffnung eine gesicherte Freispülung der seitlichen Öffnung auch bei Verklumpung der Stoffe ermöglicht wird. Folglich ermöglicht die Freispülöffnung auf jeden Fall eine gesicherte Füllung des Behältnisses mit Stoffen.

Bei einer weiteren bevorzugten Ausführungsform ist vorgesehen, dass die zumindest eine Freispülöffnung in einer parallel oder im Wesentlichen parallel zu dem Boden des Behältnisses verlaufenden, den Spalt begrenzenden bodenseitige Wandung im Wesentlichen senkrecht zum Boden des Behältnisses gerichtet angeordnet ist. Dabei kann sich die Freispülöffnung entlang einer Längsachse des Steigrohrs erstrecken und die zumindest eine seitliche Öffnung kann rechtwinklig zu der Längsachse und im Wesentlichen parallel zu dem Boden des Behälters gerichtet sein.

Durch die zum Boden gerichtete Freispülöffnung wird eine vorzugsweise "chaotische" Drehbewegung im unteren Teil des Behältnisses erzeugt. Dadurch wird erreicht, dass sämtliche bodenseitig abgelagerten Salze gelöst werden können. Somit wird auch eine Schattenbildung im Kern bzw. Zentrum des Behältnisses vermieden.

Eine weitere bevorzugte Ausführungsform zeichnet sich dadurch aus, dass der Spalt mit einer Spaltbreite A ausgebildet ist, wobei die Spaltbreite A in einem Bereich von 2 mm ≤ A ≤ 20 mm einstellbar ist, vorzugsweise A = 10 mm beträgt.

Zur Einstellung der Spaltbreite ist vorgesehen, dass der mit dem Steigrohr verbundene Schlauchanschluss als eine Gewindemuffe ausgebildet ist, mittels der das Steigrohr an einem Deckel des Behälters unter Bildung des Spaltes befestigt ist. Vorzugsweise ist die Spaltbreite A zwischen dem bodenseitigen Ende des Steigrohrs und dem Boden mittels der Gewindemuffe einstellbar.

Eine konstruktiv einfache Ausführungsform zeichnet sich dadurch aus, dass das bodenseitige Ende des Steigrohres zylinderstumpfförmig ausgebildet ist, wobei die zumindest eine seitliche Öffnung wie Bohrung in einer Seitenwandung des zylinderstumpfförmigen Endes und dass die bodenseitige Wandung mit der zumindest einen Freispülöffnung wie Bohrung als eine stirnseitige Wandung des zylinderstumpfförmigen Endes ausgebildet ist.

Um während der Produktion des Dialysekonzentrats eine effektive radiale Drehbewegung in dem Behältnis zu erreichen, ist vorgesehen, dass das Steigrohr außermittig nahe einer Außenwandung des Behälters angeordnet ist und parallel oder im Wesentlichen parallel zu einer Längsachse des Behälters verläuft und dass in einer Seitenwandung des zylinderförmigen Steigrohrs bodenseitig ein oder mehrere Öffnungen wie Bohrungen vorgesehen sind.

Gemäß einer weiteren bevorzugten Ausführungsform können in der Seitenwandung mehrere seitliche Öffnungen wie Bohrungen angeordnet sind, die in einer Umfangsrichtung des vorzugsweise zylinderförmigen Behälters ausgerichtet sind, wobei die Öffnungen in einem Winkel α mit 5° ≤ α ≤ 25° vorzugsweise α = 15° umfangsseitig verteilt angeordnet sind.

Gemäß einer weiteren bevorzugten Ausführungsform kann das zylinderstumpfförmige Ende als eine Endkappe ausgebildet sein, wobei die Endkappe mit einem stirnseitigen Ende des zylinderförmigen Steigrohrs verbunden wie verschweißt ist.

Weitere Einzelheiten, Vorteile und Merkmale der Erfindung ergeben sich nicht nur aus den Ansprüchen, den diesen zu entnehmenden Merkmalen -für sich und/oder in Kombination-, sondern auch aus der nachfolgenden Beschreibung von der Zeichnung zu entnehmenden bevorzugten Ausführungsbeispielen.

Es zeigen:
- Fig. 1: eine Draufsicht eines Behältnisses zur Herstellung eines Dialysekonzentrats,
- Fig. 2: eine Schnittdarstellung des Behältnisses entlang der Schnittlinie AA gemäß Fig. 1,
- Fig. 3: eine Seitenansicht des Steigrohrs,
- Fig. 4: eine Seitenansicht einer Endkappe des Steigrohrs,
- Fig. 5: eine Schnittdarstellung der Endkappe entlang der Schnittlinie A-A gemäß Fig. 4 und
- Fig. 6: eine Schnittdarstellung der Endkappe entlang der Schnittlinie B-B gemäß Fig. 4.

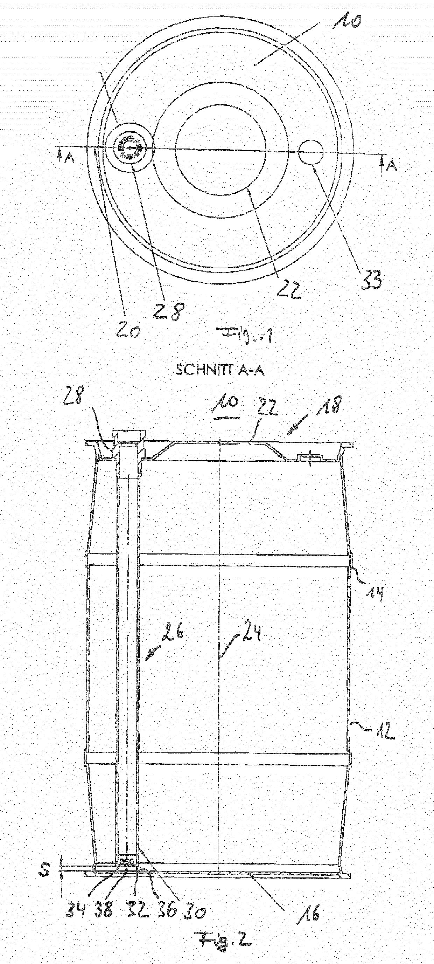

Die Fig. 1 und 2 zeigen einen Behälter 10 in Draufsicht sowie in seitlicher Schnittdarstellung in Form eines vorzugsweise wiederverwendbaren zylinderförmigen Fasses, insbesondere UC-Fass, aus einem geeigneten Material wie PE-Kunststoff. Der Behälter umfasst eine zylinderförmige Seitenwandung 12 mit Verstärkungsrippen 14, wobei die zylinderförmige Wandung 12 bodenseitig durch einen Boden 16 und oberseitig durch einen Deckel 18 begrenzt ist. Der Deckel 18 ist über eine Schweißnaht 20 mit der umlaufenden Wandung 12 verbunden und umfasst eine Befüllöffnung 22, über die zur Herstellung des Dialysekonzentrats notwendige Stoffe in den Behälter 10 eingefüllt werden können.

Von dem Deckel 18 erstreckt sich parallel oder im Wesentlichen parallel zu einer Längsachse 24 des Behälters 10 ein Steigrohr 26, welches sich von einem in dem Deckel 18 fixierten Schlauchanschluss wie Gewindemuffe 28 außermittig, d. h. angrenzend an die Wandung 12, in Richtung des Bodens 16 erstreckt. In einem bodenseitigen Ende 30 des Steigrohrs 26 sind seitliche Öffnungen 32 vorgesehen, die sich in Umfangsrichtung erstrecken und über die Flüssigkeit während der Produktion des Dialysekonzentrats aus dem Steigrohr ausströmt und in eine radiale Drehbewegung versetzt wird. Ferner ist an dem Deckel 18 ein blindlaufender Schlauchanschluss 33 vorgesehen, an dem ein freies Ende eines mit dem Schlauchanschluss 28 verbundenen Schlauches befestigt werden und am anderen Ende sich ein Filter befinden kann.

Das Steigrohr ist erfindungsgemäß so in dem Behälter 10 angeordnet, dass eine bodenseitige, stirnseitige Wandung 34 des Steigrohrs 26 unter Bildung eines vorzugsweise einstellbaren Spaltes 36 über dem Boden 16 endet, wobei in der stirnseitigen Wandung 34 eine vorzugsweise senkrecht auf den Boden 16 gerichtete Freispülöffnung 38 vorgesehen ist. Der Spalt 36 wird auf ein Spaltmaß A von vorzugsweise A = 10 mm eingestellt, um zu verhindern, dass sich die Stoffe zur Herstellung des Dialysekonzentrates in dem Spalt 36 einlagern und verklumpen. Durch den Spalt 36 wird ein Freispülkanal gebildet, der die Freispülöffnung strömungstechnisch mit der seitlichen Öffnung verbindet.

Durch die senkrecht zu dem Boden 16 gerichtete und sich in den Spalt 36 erstreckende Freispülöffnung 38 wird eine Freispülung der seitlichen Öffnungen 32 durch den Freispülkanal in dem Fall erreicht, wenn die seitliche Öffnung durch beispielsweise eine Klumpenbildung der für die Herstellung des Dialysekonzentrats in dem Behälter 10 eingefüllten Stoffe verstopft ist.

Der Erfindung liegt der Gedanke zu Grunde, dass aufgrund der geringen Spaltbildung unterhalb des Steigrohres die Stoffe nicht in einem Umfang in den Spalt 36 gelangen können, der eine Verstopfung der Freispülöffnung 38 verursachen kann. Folglich ist sichergestellt, dass Freispülöffnung 38 nicht verstopfen kann und Flüssigkeit in den Spalt 36 gelangt, über den sodann eine Freispülung der seitlichen Öffnungen 32 im Falle einer Verstopfung dieser erfolgt.

Unabhängig davon wird durch die zusätzliche Freispülöffnung eine Strömung in radialer Richtung des Behälters erreicht, wodurch bodenseitig, d. h. im unteren Teil des Behälters eine "chaotische" Drehbewegung erzeugt wird. Dadurch kann eine Schattenbildung im Kern bzw. Zentrum des Behälters vermieden werden, mit der Folge, dass sämtliche Stoffe gelöst werden können.

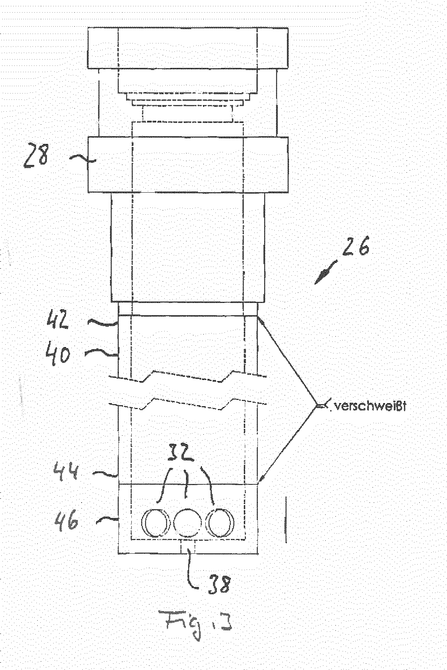

Fig. 3 zeigt eine Seitenansicht des Steigrohres. Dieses umfasst einen zylinderförmigen Rohrabschnitt 40, der mit einem oberen stirnseiteigen Ende 42 mit der Gewindemuffe 28 verbunden wie verschweißt ist. An einem bodenseitigen stirnseitigen Ende 44 ist eine Endkappe 46 verbunden wie verschweißt, in der sowohl die Freispülöffnung 38 als auch die seitlichen Öffnung 32 als vorzugsweise Bohrungen angeordnet sind.

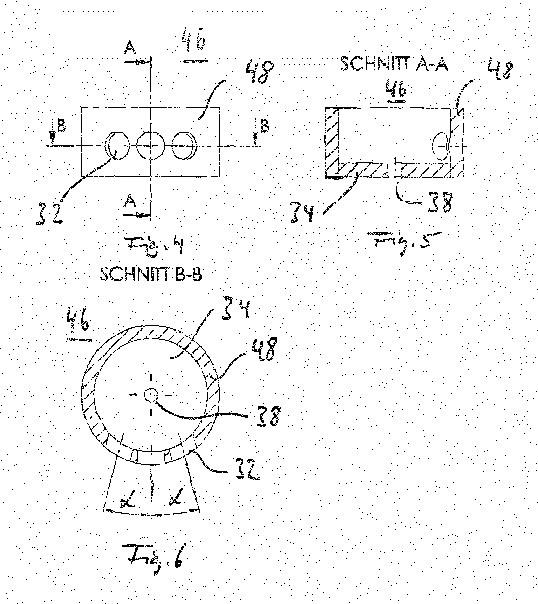

Die Endkappe 46 ist in den Fig. 4 bis 6 in verschiedenen Ansichten dargestellt. Fig. 4 zeigt eine Seitenansicht der Endkappe 46, wobei die seitlichen Öffnungen 32 in einer umlaufenden Wandung 48 der im Wesentlichen topfförmigen Endkappe als Bohrungen eingebracht sind.

Fig. 5 zeigt eine Schnittdarstellung der Endkappe 46 entlang der Schnittlinie A-A. Dabei ist die Freispülöffnung 38 in der stirnseitigen Wandung 34 zentral als Bohrung ausgebildet.

Fig. 6 zeigt eine Schnittdarstellung der Endkappe 46 entlang der Schnittlinie B-B gemäß Fig. 4, aus der zu entnehmen ist, dass die seitlichen Bohrungen 32 in Umfangsrichtung in einem Winkel α mit vorzugsweise α =15° angeordnet sind. Im dargestellten Ausführungsbeispiel weist die Endkappe 46 bzw. das Steigrohr 26 drei seitliche Öffnungen 32 auf. Die seitlichen Öffnungen 32 sind im eingebauten Zustand des Steigrohrs, wie dies in Fig. 2 in Schnittdarstellung dargestellt ist, ebenfalls in Umfangsrichtung ausgerichtet, um eine radiale Drehbewegung in der Flüssigkeit innerhalb des Behälters 10 während der der Herstellung des Dialysekonzentrats zu gewährleisten.

Durch die gleichzeitig senkrecht zum Boden 16 gerichtete Freispülöffnung 38 austretende Flüssigkeit wird eine Verwirbelung dieser unterhalb des Steigrohrs 26, d. h. eine Verwirbelung in dem Spalt 36 erreicht, wodurch bodenseitig eine "chaotische" Drehbewegung zur Vermeidung einer Schattenbildung im Zentrum des Behälters 10 erreicht wird.

## Patentansprüche

1. Behälter (10), insbesondere wiederverwendbaren Mischbehälter, zur Herstellung von Dialysekonzentraten, wobei der Behälter (10) zur Herstellung eines Dialysekonzentrates benötigte Stoffe mit Ausnahme von Wasser enthält, umfassend ein vorzugsweise integriertes Steigrohr (26) zum Zuführen und Absaugen von Flüssigkeit, wobei sich das Steigrohr (26) von einem Schlauchanschluss (28) bis zu einem Boden (16) des Behälters (10) erstreckt und zumindest eine seitliche Öffnung (32) in einem bodenseitigen Ende (30) aufweist,
**dadurch gekennzeichnet ,**
**dass** das Steigrohr (26) zumindest eine Freispülöffnung (38) zur Erzeugung einer Flüssigkeitsströmung zur Auflösung seiner die zumindest eine seitliche Öffnung verstopfenden Verklumpung von Stoffen aufweist.

2. Behälter nach Anspruch 1,
**dadurch gekennzeichnet,**
**dass** die zumindest eine Freispülöffnung (38) in dem bodenseitigen Ende (30) angeordnet und in einen zwischen bodenseitigem Ende (30) und dem Boden (16) gebildeten Freiraum (36) ausgerichtet ist.

3. Behälter nach Anspruch 1 oder 2,
**dadurch gekennzeichnet,**
**dass** das bodenseitige Ende (30) unter Bildung des Freiraums in Form eines Spaltes (36) über dem Boden (16) angeordnet ist und dass die Freispülöffnung (38) in den zwischen dem bodenseitigen Ende (30) und dem Boden (16) gebildeten, strömungstechnisch mit der zumindest einen seitlichen Öffnung (32) gekoppelten Spalt (36) als Freispülkanal gerichtet ist.

4. Behälter nach zumindest einem der vorhergehenden Ansprüche,
**dadurch gekennzeichnet ,**
**dass** die zumindest eine Freispülöffnung (38) in einer parallel oder im Wesentlichen parallel zu dem Boden (16) des Behältnisses verlaufenden, den Spalt (36) begrenzenden bodenseitige Wandung (34) im wesentlichen senkrecht zum Boden (16) des Behältnisses (10) gerichtet angeordnet ist.

5. Behälter nach zumindest einem der vorhergehenden Ansprüche,
**dadurch gekennzeichnet ,**
**dass** der Spalt (36) mit einer Spaltbreite A ausgebildet ist, wobei die Spaltbreite A in einem Bereich von 2 mm ≤ A ≤ 20 mm einstellbar ist, vorzugsweise A = 10 mm, beträgt.

6. Behälter nach zumindest einem der vorhergehenden Ansprüche,
**dadurch gekennzeichnet ,**
**dass** der mit dem Steigrohr (26) verbundene Schlauchanschluss (28) als eine Gewindemuffe ausgebildet ist, mittels der das Steigrohr (26) an einem Deckel (22) des Behälters (10) unter Bildung des Spaltes (36) befestigt ist.

7. Behälter nach zumindest einem der vorhergehenden Ansprüche,
**dadurch gekennzeichnet ,**
**dass** die Spaltbreite A zwischen dem bodenseitigen Ende (34) des Steigrohrs (26) und dem Boden (16) mittels der Gewindemuffe (28) einstellbar ist.

8. Behälter nach zumindest einem der vorhergehenden Ansprüche,
**dadurch gekennzeichnet ,**
**dass** das bodenseitige Ende (30) des Steigrohres (26) zylinderstumpfförmig ausgebildet ist, wobei die zumindest eine seitliche Öffnung (32) wie Bohrung in einer Seitenwandung des zylinderstumpfförmigen Endes (30) und dass die bodenseitige Wandung (34) mit der zumindest einen Freispülöffnung (38) wie Bohrung als eine stirnseitige Wandung des zylinderstumpfförmigen Endes (30) ausgebildet ist.

9. Behälter nach zumindest einem der vorhergehenden Ansprüche,
**dadurch gekennzeichnet ,**
**dass** in der Seitenwandung mehrere seitliche Öffnungen (32) wie Bohrungen angeordnet sind, die in einer Umfangsrichtung des vorzugsweise zylinderförmigen Behälters (10) ausgerichtet sind, wobei die Öffnungen (32) in einem Winkel α mit 5° ≤ α ≤ 25° vorzugsweise α = 15° umfangsseitig verteilt angeordnet sind.

10. Behälter nach zumindest einem der vorhergehenden Ansprüche,
**dadurch gekennzeichnet ,**
**dass** das zylinderstumpfförmige Ende (30) als eine Endkappe (46) ausgebildet ist, wobei die Endkappe (46) mit einem stirnseitigen Ende (44) des zylinderförmigen Steigrohrs (26) verbunden wie verschweißt ist.

11. Behälter nach zumindest einem der vorhergehenden Ansprüche,
**dadurch gekennzeichnet ,**
**dass** die Freispülöffnung (38) sich entlang einer Längsachse des Steigrohrs (26) erstreckt und dass die zumindest eine seitliche Öffnung (32) rechtwinklig zu der Längsachse und im Wesentlichen parallel zu dem Boden (16) des Behälters gerichtet ist.

12. Behälter nach zumindest einem der vorhergehenden Ansprüche,
**dadurch gekennzeichnet ,**
**dass** das Steigrohr (26) außermittig nahe einer Außenwandung (12) des Behälters (10) angeordnet ist und parallel oder im Wesentlichen parallel zu einer Längsachse (24) des Behälters (10) verläuft.
